# EUROPÄISCHE PATENTSCHRIFT

(11) **EP 3 815 642 B1**
(45) Veröffentlichungstag und Bekanntmachung des Hinweises auf die Patenterteilung: **07.06.2023**
(21) Anmeldenummer: 19205674.5
(22) Anmeldetag: 28.10.2019
(51) Int. Cl.: A61B 18/14, A61B 17/32, A61B 17/295

(54) **MESSERKARTUSCHE UND VERSIEGELUNGSELEMENT**
BLADE CARTRIDGE AND SEALING INSTRUMENT
CARTOUCHE DE LAME ET INSTRUMENT POUR SCELLER

(43) Veröffentlichungstag der Anmeldung: 05.05.2021
(73) Patentinhaber: Erbe Elektromedizin GmbH, 72072 Tübingen (DE)
(72) Erfinder: BOB, Felix, 72108 Rottenburg (DE); KAUPP, Stefan, 72072 Tuebingen (DE); BAUER, Linda, 72108 Rottenburg (DE); BREUNINGER, Tobias, Dr., 72585 Riederich (DE); LEHMANN, Bjoern, Dr., 72116 Moessingen (DE)
(74) Vertreter: Rüger Abel Patentanwälte PartGmbB

(56) Entgegenhaltungen:
- EP-A1- 1 609 430
- US-A1- 2003 220 637
- US-A1- 2010 292 691
- US-A1- 2011 004 208
- US-A1- 2017 333 037

## Beschreibung

Die Erfindung betrifft eine Messerkartusche und ein Versiegelungsinstrument, insbesondere zur Versiegelung und Durchtrennung von Gefäßen.

Versiegelungsinstrumente werden in der Chirurgie typischerweise verwendet, um Gefäße, beispielsweise Blutgefäße, Lymphgefäße, andere Gefäße oder sonstige Gewebe zu versiegeln und zu durchtrennen. Beim Versiegeln wird ein dauerhafter Gefäßverschluss ausgebildet, wonach das Gefäß an der geschlossenen Stelle getrennt wird.

Dazu ist aus der US 2011/004208 A1 ein Versiegelungsinstrument mit zwei Branchen bekannt, die an ihren jeweiligen proximalen Enden Handgriffe aufweisen. Die beiden Branchen sind gelenkig aneinander gelagert und bilden mit ihren distalen Enden ein Versiegelungswerkzeug. An einer der Branchen ist ein in distaler Richtung verschiebbar gelagertes Messer angeordnet, das in einem Schlitz der Branche geführt ist und zum Durchtrennen eines Gefäßes dient. Zur Versiegelung des Gefäßes können die Gewebeanlageflächen der distalen Teile der beiden Branchen bestromt werden, um das zwischen den geschlossenen Branchen gehaltene Gefäß zu fusionieren.

Das Instrument kann so lange gebraucht werden bis das Messer abgestumpft ist und keinen sauberen Schnitt mehr erbringt. Danach muss zumindest das Messer ausgetauscht werden. Dazu ist das Messer in einem Wechselteil aus Kunststoff untergebracht, das das verschiebbare Messerelement und Versiegelungselektroden trägt, die an länglichen mit den distalen Teilen der Branchen zu verbindenden Abschnitten des Wechselwerkzeugs verbunden sind. Die beiden länglichen Abschnitte sind über ein Federgelenk miteinander verbunden, sodass sie die Bewegung der Branchen nachvollziehen können. Das Wechselwerkzeug weist ein elektrisches Anschlusskabel auf, das mit einer Branche des Instruments verbunden ist.

Aus der US 2003/220637 A1 ist ein Versiegelungsinstrument mit zwei gelenkig aneinander gelagerten Branchen bekannt, die zur elektrischen Versiegelung von Gefäßen an ihren distalen Abschnitten entsprechende Versiegelungselektroden aufweisen. Außerdem weist das Instrument ein auswechselbar gehaltenes Messer auf, das mit einer der Branchen rastend verbunden und dann distal verschiebbar gelagert ist.

Die US 2013/046303 A1 offenbart ein Instrument mit Versiegelungselektroden und einem mechanischen Messer, das in einem Messerkanal verschiebbar angeordnet ist. Anstelle des mechanisch beweglichen Messers kann in eine entsprechende Aufnahme der Branchen eine elektrische Schneide eingesetzt werden.

Aus der WO 2018/078646 A1 geht ähnlich wie aus der US 2011/004208 A1 ein Instrument hervor, bei dem ein Wechselwerkzeug vorgesehen ist, das außerdem die Versiegelungselektroden enthält und insgesamt auswechselbar an dem Instrument angebracht ist.

Ein weiteres Instrument mit austauschbarem Messer geht aus der EP 2 732 779 A1 hervor.

Die US 5,913,874 A beschreibt ein Versiegelungsinstrument mit einem wiederverwendbaren Grundkörper und einer austauschbaren Kartusche, die sowohl das Messer als auch die Versiegelungselektroden enthält.

Weiterer Stand der Technik geht aus der US 2013/085516 A1 der US 2016/206366 A1**,** der EP 2 245 993 A2**,** der EP 2 086 440 A1**,** der US 2010/179545 A1**,** der US 8,685,056 B2**,** der US 9,717,521**,** der EP 2 489 320 A2**,** der US 2010/0292691 A1**,** der EP 1 609 430 A1 und der DE 196 37 133 A1 hervor.

Es ist Aufgabe der Erfindung, ein Versiegelungsinstrument mit auswechselbarem Messer zu gestalten, bei dem der Messerwechsel einfach und sicher durchführbar ist. Insbesondere soll eine Gefährdung des beteiligten Personals ausgeschlossen sein.

Diese Aufgabe wird mit dem erfindungsgemäßen Versiegelungsinstrument mit der zugehörigen Messerkartusche gelöst:

Das erfindungsgemäße Versiegelungsinstrument kann insbesondere zur Gefäßversiegelung und dabei insbesondere für den offenchirurgischen Einsatz eingerichtet sein. Es weist eine erste Branche auf, die an ihrem proximalen Ende einen Handgriff aufweist oder über einen Schaft mit einem solchen verbunden ist. An ihrem distalen Ende weist sie ein erstes Werkzeugteil eines Versiegelungswerkzeugs z.B. in Gestalt einer oder mehrerer Versiegelungselektroden auf. Es ist eine zweite Branche vorgesehen, die die an ihrem distalen Ende ein zweites Werkzeugteil, z.B. wiederum eine oder mehrere Versiegelungselektroden, aufweist und in Bezug auf die erste Branche schwenkbar gelagert und dazu beispielweise schwenkbar mit dieser verbunden ist. In der Nähe des Werkzeugs ist ein Kartuschensitz zur Aufnahme einer Messerkartusche vorgesehen.

Die Messerkartusche weist ein Gehäuse auf, das einen Innenraum umschließt, in dem ein Messer angeordnet ist. Es kann dabei ein Verbindungsmittel vorgesehen sein, mittels dessen die Messerkartusche an dem Kartuschensitz arretierbar ist. Zumindest bei einem scherenartig ausgebildeten Instrument mit zwei gelenkig miteinander verbundenen Branchen und Scherengriffen am jeweiligen distalen Ende derselben kann der Kartuschensitz an einer der Branchen ausgebildet sein.

Das Messer weist mindestens eine Schneidkante auf und kann in die Messerkartusche eingezogen und aus dieser herausgeschoben werden. Dazu kann die Messerkertusche eine Betätigungseinrichtung aufweisen. Alternativ kann eine Kupplungseinrichtung zum Anschluss des Messers an eine Betätigungseinrichtung vorgesehen sein. Das Messer ist, wenn die Kartusche nicht in das Versiegelungsinstrument eingesetzt ist, im Innenraum der Kartusche gehalten. Wenn die Messerkartusche an dem Kartuschensitz angebracht ist, kann das Messer mittels der Betätigungseinrichtung wenigstens so weit aus der Messerkartusche heraus bewegt werden, wie es für die Durchführung eines Schnitts an Gewebe erforderlich ist, das von dem Instrument gefasst ist.

Der Messerwechsel erfolgt, indem eine gebrauchte Messerkartusche von dem Kartuschensitz abgenommen und eine neue Messerkartusche an diesem angesetzt und dort befestigt wird. Dazu können Mittel vorgesehen sein, die die Kartusche am Kartuschensitz halten, diese jedoch freigeben, wenn die Schenkel des Instruments in Reinigungsposition überführt werden. Die Reinigungsposition, in der die Kartusche freigegeben ist, unterscheidet sich von den anderen Positionen, d.h. den Gebrauchspositionen, darin, dass die beiden mit den Griffen versehenen Schenkel weiter geöffnet sind, als beim Einsatz des Instruments am Patienten nötig oder üblich. Dadurch wird verhindert, dass die als Einmal-Teil ausgebildete Kartusche während der Aufbereitung des Instruments an diesem verbleibt.

Mit dem erfindungsgemäßen Konzept wird nicht nur die Verletzungsgefahr für das beteiligte Personal gemindert oder beseitigt, es wird zugleich eine konstruktiv und für Reinigungsaspekte vorteilhafte Aufteilung von Einwegmaterial in Gestalt der Messerkartusche und sterilisierbarem Instrumententeil geschaffen. Keinem oder nur geringem Verschleiß unterliegende Teile sind Bestandteil des Instruments. Dies betrifft neben den Branchen und dem Gelenk insbesondere die Versiegelungselektroden. Die Unterbringung des Messers in einer Messerkartusche schützt nicht nur vor Verletzungen, erleichtert die Handhabung und ermöglicht einen schnelleren Messerwechsel (Einlegen u. Ausgeben), sondern es wird zugleich eine übersichtliche glattflächige, leicht zu reinigende Schnittstelle in Gestalt des Kartuschensitzes geschaffen. Zusätzlich können gegenüber einem kompletten Einmal-Instrument technischer Aufwand zur Bereitstellung von Instrumenten gesenkt und Ressourcen eingespart werden.

Bei einer bevorzugten Ausführungsform weist die Messerkartusche einen Verriegelungsmechanismus zur Arretierung des Messers in einer ersten Position auf, in der sich die Schneide des Messers in dem Innenraum des Gehäuses der Kartusche befindet. Eine solche Messerkartusche ist sicher handhabbar und es sind Stich- oder Schnittverletzungen des damit umgehenden Personals ausgeschlossen.

Der Verriegelungsmechanismus umfasst, zumindest bei einer möglichen Ausführungsform, ein an dem Gehäuse der Messerkartusche beweglich gelagertes Sperrglied, dem eine an dem Messer ausgebildete Sperrstruktur zugeordnet ist. Das Sperrglied kann ein verschiebbares Sperrglied oder ein schwenkbar gelagertes, gesondertes oder als Teil der Kartusche ausgebildetes Sperrglied sein, das wenigstens einen mit der Sperrstruktur des Messers zusammenwirkenden Abschnitt aufweist. Die Sperrstruktur des Messers kann eine Ausnehmung, ein Vorsprung oder ähnliches sein. Beispielsweise kann das Sperrglied ein Sperrstift oder ein Sperrbolzen sein, der, um eine Bewegung des Messers zu sperren, sich in oder durch eine Ausnehmung des Messers erstreckt. Der Sperrstift oder Sperrbolzen kann beweglich gelagert sein, um mit der Sperrstruktur des Messers in und außer Eingriff überführt zu werden. Beispielsweise kann der Sperrstift dazu quer zur Bewegungsrichtung des Messers beweglich gelagert sein und eine Ausnehmung aufweisen, durch die das Messer hindurch bewegbar ist. Fluchtet die Ausnehmung mit dem Messer, ist die Längsverschiebebewegung des Messers freigegeben. Ist der Sperrstift jedoch aus dieser Freigabeposition herausverschoben, durchgreift er eine Sperrstruktur, beispielweise eine Einkerbung des Messers, und sperrt somit dessen Längsbewegung. Das Sperrglied kann aber auch als einstückiger Bestandteil der Kartusche ausgebildet sein, z.B. als federnde Rastzunge oder ähnliches.

Das Sperrglied kann auch schwenkbar gelagert sein, um mit einer Sperrstruktur des Messers in und außer Eingriff gebracht zu werden.

Vorzugsweise ist auch eine Verriegelungseinrichtung zur Arretierung der Messerkartusche an dem Kartuschensitz vorgesehen. Diese Verriegelungseinrichtung kann als Teil des Verriegelungsmechanismus zur Arretierung der Messerkartusche an dem Kartuschensitz ausgebildet sein. Dazu kann das Sperrglied mit einer entsprechenden Gegenfläche des Kartuschensitzes zusammenwirkend ausgebildet sein, um das Messer beim Einsetzen der Messerkartusche in den Kartuschensitz zu entsperren und das Messer beim Herausnehmen der Messerkartusche aus dem Instrument in seiner sicheren Position zu arretieren.

Der Verriegelungsmechanismus kann insbesondere eine Wechselsperre umfassen, die in einer ersten Stellung das Messer mit seiner Schneide in der Messerkartusche hält und in einer zweiten Stellung, in der das Messer freigegeben ist, die Messerkartusche an dem Kartuschensitz verriegelt. Eine solche Wechselsperre kann beispielsweise durch ein verschiebbar oder schwenkbar gelagertes Sperrglied gebildet sein, das entweder in die Sperrstruktur des Messers oder in eine an dem Kartuschensitz ausgebildete Sperrstruktur eingreift. Die Hin- und Herbewegung des Sperrglieds kann durch das Ansetzen der Messerkartusche an den Kartuschensitz und das Abnehmen der Messerkartusche von dem Kartuschensitz gesteuert werden. Das Lösen der durch die Wechselsperre in dem Instrument verriegelten Messerkartusche kann durch eine Bewegung der beiden Schenkel des Instruments z.B. in eine überweit geöffnete Position bewirkt werden, die bei normalem Einsatz am Patienten nicht eingenommen wird.

Ist die Sperrstruktur des Messers eine Ausnehmung, in die das Sperrglied durch eine Bewegung quer zur Bewegungsrichtung des Messers eingreift, kann das Sperrglied nur dann in seine Messersperrposition überführt werden, wenn das Messer in sicherer Position steht, d.h., die Sperrstruktur mit dem Sperrglied fluchtet. In dieser Position befindet sich die Schneide des Messers in sicherer Position innerhalb des Innenraums der Messerkartusche. Weil das Sperrglied nur in dieser Position in die Sperrstruktur des Messers einfallen kann, kann es auch nur in dieser Position die Messerkartusche zur Herausnahme derselben aus dem Kartuschensitz freigeben.

Damit ist Sicherheit für das Bedienpersonal sowohl beim Einsetzen der Messerkartusche in das Instrument als auch beim Herausnehmen der Messerkartusche aus dem Instrument gegeben.

Die Messerkartusche weist vorzugsweise einen Messerkanal auf, der das Messer in der Kartusche längsbeweglich führt. Dieser Messerkanal ist vorzugsweise fluchtend mit einem an der Branche vorgesehenen Messerkanal ausgerichtet, wenn die Kartusche an dem Kartuschensitz befestigt ist. Der proximale Teil des Messerkanals stellt an einem Instrument normalerweise den am schwierigsten zu reinigende Teil des Messerkanals dar. Der Teil des Messerkanals, in dem sich das Messer in Ruheposition befindet, ist bei der Sterilisation besonders schwer zugänglich. Das Messer transportiert bei Benutzung jedoch Schmutz an diese Position. Dieser Teil des Messerkanals ist nun Teil der Kartusche und wird mit dieser ausgetauscht. In den Versiegelungsbacken ist der Kanal hingegen zu einer Seite hin offen und auch im Gelenkbereich können Reinigungsöffnungen vorgesehen sein.

Weil zumindest bei der bevorzugten Ausführung zugleich mit dem Ansetzen der Kartusche an den Kartuschensitz das Messer in der Messerkartusche entriegelt wird, kann das Messer, dessen proximales stumpfes Ende aus der Kartusche herausschauen kann, einfach in eine Schiebebewegung in den Messerkanal des Instruments eingeführt werden. Dabei kann eine Betätigungseinrichtung mit einer entsprechenden Kupplungsstruktur des Messers in Eingriff kommen. Die Kupplungsstruktur kann zum Beispiel eine Ausnehmung sein, in die ein Mitnehmer der Betätigungseinrichtung einrastet, sobald diese Ausnehmung in den Bereich der Betätigungseinrichtung geschoben ist.

Auch kann bei einer Ausführung der Erfindung das Messer im ausgebauten Zustand komplett in der Kartusche positioniert sein. Bei einer solchen Ausführungsform ist die Handhabung besonders einfach und sicher.

An dem insoweit beschriebenen Instrument sind viele Abwandlungen möglich. Beispielsweise kann das Messer in der Messerkartusche auf seine sichere Position hin vorgespannt gehalten sein, wobei die Schneide des Messers in der sicheren Position im Innenraum des Gehäuses der Messerkartusche steht. Zur Vorspannung auf die sichere Position hin, kann ein Federelement zum Beispiel in Gestalt einer Druckfeder, eine Zugfeder, ein Magnet oder dergleichen vorgesehen sein.

Auch ist es möglich, die Messerkartusche und die Betätigungseinrichtung zu einer von dem Instrument insgesamt entfernbaren Kartusche zu vereinigen.

Weiter ist es möglich, die Messerkartusche mit der Betätigungseinrichtung und einem Handgriff zu vereinigen, sodass diese erweiterte Messerkartusche den proximalen Teil einer Branche bildet.

Weiter ist es vorteilhaft, wenn die Werkzeugteile des Versiegelungswerkzeugs mit den Branchen fest verbunden sind. Die Messerkartusche ist dann das einzige leicht von dem Instrument lösbare Teil, was Verwechselungen und Fehlbedienungen vermeidet. Insbesondere sind die Messerkartusche und die Werkzeugteile voneinander getrennte, nicht fest miteinander verbundene Baugruppen.

Es sind weitere Möglichkeiten der Positionierung der Kartusche gegeben. Z.B. können der Kartuschensitz und somit die Kartusche im Gelenkbereich des Instruments angeordnet sein. Durch die Nähe der Kartusche zu dem Klemmbereich des Instruments, in dem das biologische Gewebe gefasst wird, sind der Verfahrweg und/oder die Länge des Messers gering. Dadurch kann die Kartusche miniaturisiert werden.

Allen Ausführungsformen ist jedoch gemeinsam, dass zur sicheren Arretierung der Messerkartusche an dem Instrument entsprechende Arretiermittel vorgesehen sind, beispielsweise in Gestalt von Rastmitteln. Diese sind vorzugsweise im Bereich des distalen Endes des Kartuschensitzes angeordnet, um die Handhabung der Kartusche beim Kartuschenwechsel zu erleichtern. Vorzugsweise ist die Bewegungsrichtung beim Anfügen der Messerkartusche an die Branche sowie beim Trennen der Messerkartusche von der Branche quer zu der Bewegungsrichtung des Messers orientiert. Es sind jedoch auch abgewandelte Ausführungsformen möglich, bei denen die Messerkartusche in einer schräg oder parallel zur Bewegungsrichtung des Messers gerichteten Bewegung an den Kartuschensitz angesetzt wird.

Weitere Details von Ausführungsformen der Erfindung sind Gegenstand der Unteransprüche, der Zeichnung oder der Beschreibung.

Es zeigen:
Figur 1 das erfindungsgemäße Versiegelungsinstrument mit Messerkartusche, in schematisierter perspektivischer Darstellung,
Figur 2, 2a eine Messerkartusche und den zugeordneten Kartuschensitz, in vereinfachter Prinzipdarstellung, in perspektivischer Seitenansicht,
Figur 3, 3a den Kartuschensitz einer Branche und die Messerkartusche in ausgerastetem Zustand, in vereinfachter Querschnittsdarstellung,
Figur 4, 4a den Kartuschensitz und die Kartusche in eingerastetem Zustand, in Querschnittsdarstellung entsprechend Figur 3,
Figur 5 ein Versiegelungsinstrument mit zugehöriger Messerkartusche in ausschnittsweiser perspektivischer Explosionsdarstellung,
Figur 6a-c die Messerkartusche für das Instrument nach Figur 5 mit Wechselsperre in verschiedenen Positionen, jeweils in Schnittdarstellung,
Figur 7 bis 9 abgewandelte Ausführungsformen des erfindungsgemäßen Versiegelungsinstruments.

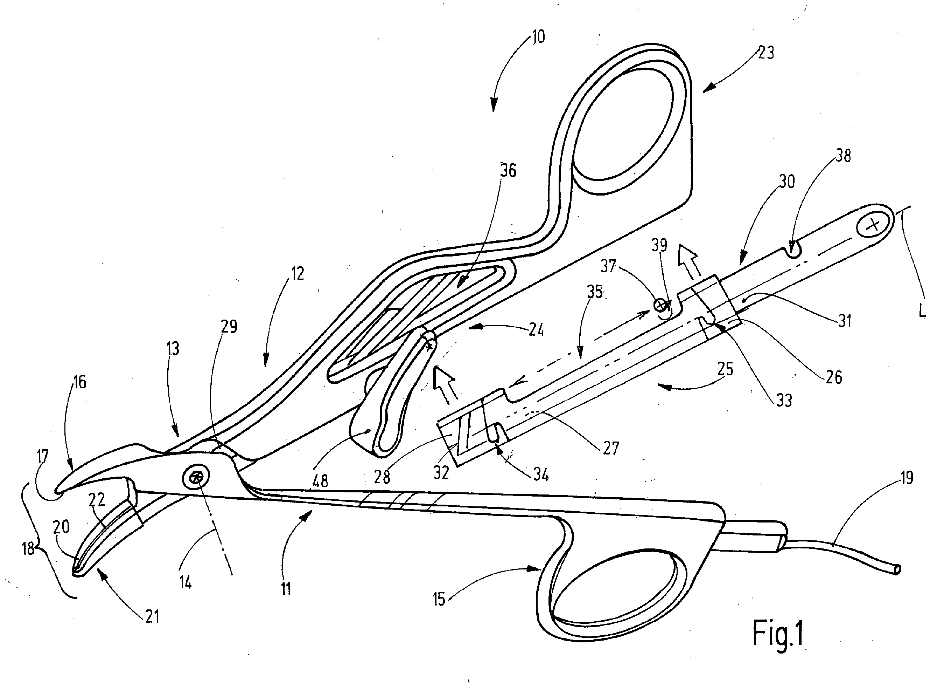

In Figur 1 ist ein Versiegelungselement 10 veranschaulicht, das eine erste Branche 11 und eine zweite Branche 12 aufweist, die mittels eines Gelenks 13 relativ zueinander schwenkbar gelagert sind. Beispielsweise kann das Gelenk 13 eine quer zu den Branchen 11, 12 orientierte Schwenkachse 14 festlegen.

Die erste Branche 11 weist einen Schenkel auf, der an seinem proximalen Ende einen Handgriff 15 trägt. Das distale und somit aus Sicht des Anwenders jenseits der Achse 14 liegende Ende 16 der Branche 11 enthält ein erstes Werkzeugteil 17, das zu einem Versiegelungswerkzeug 18 gehört. Der erste Werkzeugteil 17 umfasst dazu mindestens eine Versiegelungselektrode, die über ein Kabel 19 mit Strom versorgbar ist, das sich von dem proximalen Ende der ersten Branche 11 weg zu einem nicht weiter veranschaulichten Gerät erstreckt.

Zu dem Versiegelungswerkzeug 18 gehört außerdem ein zweiter Werkzeugträger 20, der durch eine an dem distalen Ende 21 der zweiten Branche 12 angebrachte Versiegelungselektrode 20 gebildet ist. Der zweite Werkzeugteil 20 weist wie auch der erste Werkzeugteil 17 einen Schlitz 22 auf, längs dessen sich ein Messer bewegen kann, um ein zwischen den Werkzeugteilen 17, 20 gefasstes und mittels Stromeinwirkung versiegeltes Gefäß oder sonstiges Gewebe zu durchtrennen. Die zweite Branche 12 weist an ihrem proximalen Ende einen Handgriff 23 auf, so dass das Instrument 10 nach Art einer Schere oder Zange handhabbar ist.

In dem sich zwischen dem Gelenk 13 und dem Handgriff 23 befindenden Abschnitt der zweiten Branche 12 ist ein Kartuschensitz 24 ausgebildet, der zur Aufnahme einer Messerkartusche 25 dient.

Die Messerkartusche 25 weist ein Gehäuse 26 auf, das einen Innenraum 27 umschließt. Das Gehäuse 26 kann eine im Wesentlichen länglich quaderförmige Grundform aufweisen, durch die sich längs ein Messerkanal 28 hindurch erstreckt. Dieser Messerkanal 28 ist vorzugsweise so angeordnet, dass er mit einem an der zweiten Branche 12 vorgesehenen Messerkanal 29 fluchtet.

In dem Innenraum 27 ist ein Messer 30 längs verschiebbar angeordnet, wobei ein stumpfer Abschnitt 31 aus dem Gehäuse 26 herausragen kann. An dem in dem Gehäuse 26 befindlichen Teil des Messers 30 ist eine Schneide 32 ausgebildet, die sich zum Beispiel quer zur Längsrichtung L des Messers 30 erstrecken kann. Die Längsrichtung L ist zugleich die Bewegungsrichtung des Messers 30.

Das Gehäuse 26 ist vorzugsweise mit Befestigungsmitteln, beispielsweise Rastmitteln 33, 34, versehen, denen entsprechende an dem Kartuschensitz 24 ausgebildete, in Figur 1 nicht weiter veranschaulichte Gegenstrukturen zugeordnet sind. Die Rastmittel 33, 34 können beispielsweise an dem Kunststoffgehäuse 26 ausgebildete Rastnasen, Rastzungen, Rastfedern oder sonstige nachgiebige Elemente sein.

Das Gehäuse 26 kann außerdem mit einem Ausschnitt 35 versehen sein, um dem Abtrieb einer Betätigungsvorrichtung 36 Zugang zu dem Messer 36 zu gewähren. Der Abtrieb der Betätigungsvorrichtung kann zum Beispiel ein Mitnehmer 37 sein, der in Figur 1 symbolisch gesondert veranschaulicht ist, der in eine Ausnehmung 38 des Messers 30 einfahren kann. Das Messer 30 kann außerdem weitere Ausnehmungen, zum Beispiel eine Ausnehmung 39, aufweisen, die zum Sperren des Messers 30 in einer Sicherheitsposition dienen. Die Sicherheitsposition ist in Figur 1 dargestellt. In dieser liegt die Schneide 32 in dem Innenraum 27 des Gehäuses 26.

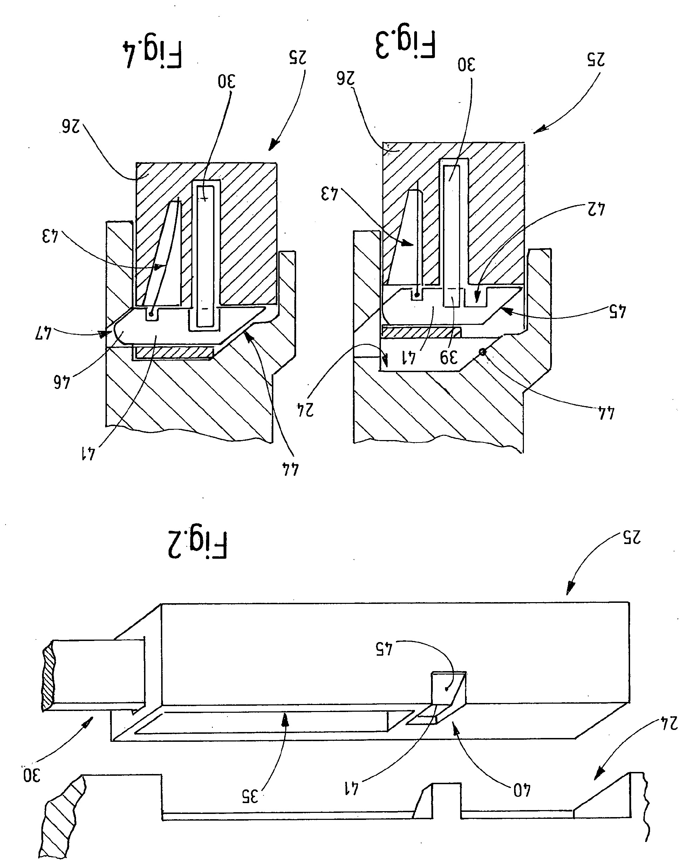

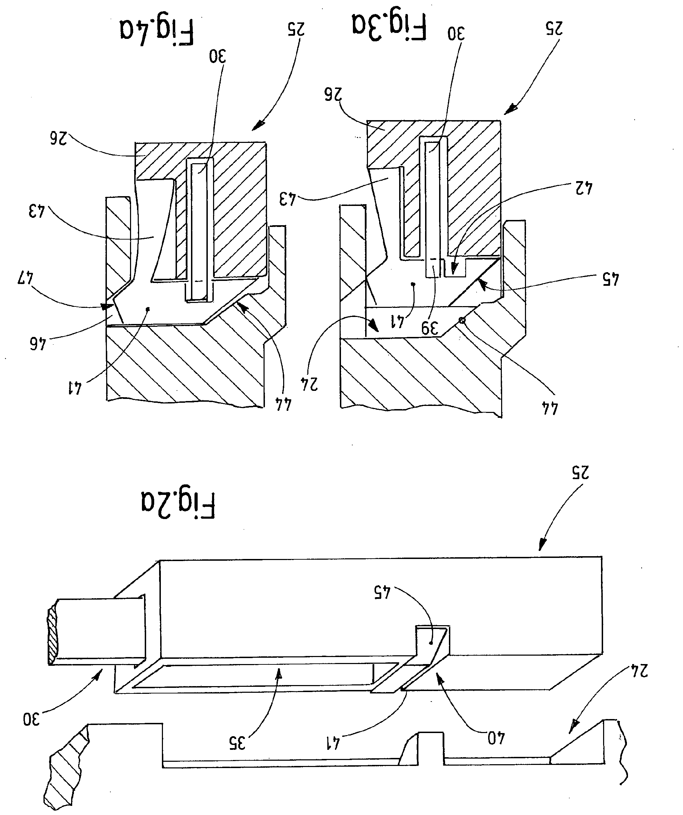

Zum Arretieren des Messers 30 in der Sicherheitsposition kann ein Verriegelungsmechanismus 40 vorgesehen sein, der dazu eingerichtet ist, das Messer 30 in der sicheren Position zu arretieren, solange die Messerkartusche 25 nicht an dem Kartuschensitz 24 befestigt ist. Vorzugsweise ist der Verriegelungsmechanismus 40 zusätzlich so ausgebildet, dass er die Messerkartusche 25 an dem Kartuschensitz 24 zumindest so lange fest verriegelt hält, wie sich das Messer 30 nicht in seiner sicheren Position befindet. Dazu kann ein bewegliches Sperrglied 41 vorgesehen sein, das der Messerkartusche 25 zugeordnet ist. Beispielsweise kann das Sperrglied 41 ein quer zur Bewegungsrichtung L des Messers 30 verschiebbarer Sperrriegel oder ein Sperrstift sein, der die Ausnehmung 39 in dem Messer 30 durchsetzt (Figur 1 und Figur 3). Alternativ kann das Sperrglied mit der Messerkartusche 25 beispielweise als schwenkbar gelagerte Zunge einteilig ausgebildet sein. Das schwenkbare Ende der Zunge kann dann als Sperrnase dienen.

Das Sperrglied 41 kann eine Ausnehmung 42 aufweisen, die durch entsprechend Bewegung des Sperrglieds 41 mit dem Messer 30 in fluchtende Übereinstimmung gebracht werden kann, wie es in Figur 4 dargestellt ist. In dieser Position gibt das Sperrglied 41 eine Längsbewegung des Messers 30 frei. Zur sicheren Sperrung des Messers 30 in sicherer Position bei aus dem Instrument 10 herausgenommener Messerkartusche 25 kann das Sperrglied 41 durch ein Federmittel 43 auf seine in Figur 3 veranschaulichte Sperrposition hin vorgespannt sein. Im Falle der einteiligen Ausbildung der Messerkartusche 25 und des Sperrgliedes 41 kann die Zunge selbst das Federmittel bilden.

An den Kartuschensitz 24 kann außerdem ein Entriegelungselement 44, beispielsweise in Gestalt einer Schrägfläche oder eines Vorsprungs, angeordnet sein, wobei dem Entriegelungselement 44 eine an dem Sperrglied 41 vorgesehene Betätigungsfläche 45 zugeordnet ist. Die Betätigungsfläche 45 kann beispielsweise schräg zur Bewegungsrichtung des Sperrglieds 41 angeordnet sein.

Das Sperrglied 41 kann außerdem eine Sperrnase 46 aufweisen, die, sobald das Messer 30 entsperrt ist (d.h., wenn die Ausnehmung 42 mit dem Messer 30 fluchtet, wie es in Figur 4 veranschaulicht ist) in eine an dem Kartuschensitz 24 vorgesehene Sperröffnung 47 greift und dadurch die Messerkartusche 25 an dem Kartuschensitz 24 verriegelt.

Die Figuren 2a, 3a und 4a veranschaulichen ein Versiegelungsinstrument 10 und eine Messerkartusche 25 in abgewandelter Ausführungsform, für die die Beschreibung der Ausführungsform nach den Figuren 2 bis 4 entsprechend gilt. Jedoch ist das Federmittel 43 bei dieser Messerkartusche 25 sowohl mit dem Sperrglied 41 und/oder dem Gehäuse 26 einstückig ausgebildet.

Das insoweit beschriebene Instrument 19 und die zugehörige Messerkartusche 25 arbeiten wie folgt:
Zur Verdeutlichung wird zunächst angenommen, dass das Instrument 10 und die zugehörige Messerkartusche 25 gesondert bereit liegen. Die Messerkartusche 25 enthält das Messer 30 in sicherer Position, in der das Messer 30 vorzugsweise verriegelt ist. Damit ist die Messerkartusche 25 handhabungssicher. Aus dem Gehäuse 26 ragt allenfalls der stumpfe Abschnitt 31, wohingegen die Schneide 32 sicher in dem Gehäuse 26 gehalten ist.

Zur Inbetriebnahme des Instruments 10 wird die Messerkartusche 25 an den Kartuschensitz 24 geführt und dort angesetzt. Dabei wird das Messer 30 durch entsprechende Bewegung des Sperrglieds 41 entriegelt, d.h., von der in Figur 3 veranschaulichten Position in die Position 4 überführt. Dabei wird zugleich die Messerkartusche 25 an dem Kartuschensitz 24 verriegelt. Die Längsbewegung des Messers ist nun aber freigegeben. Dadurch kann der Bediener das Messer 30 an dem stumpfen Teil 31 fassen und durch den Messerkanal 28 in Figur 1 nach links vorwärts schieben, so dass die Schneide 32 in den Messerkanal 29 übertritt. Dabei kann zugleich die Ausnehmung 38 in dem Bereich des Mitnehmers 37 geraten, wodurch das Messer 30 an die Betätigungseinrichtung 36 ankuppelt. Das Messer 30 sperrt in dieser Position eine Rückbewegung des Sperrglieds 41, sodass, sobald das Messer 30 so verschoben ist, dass seine Schneide 32 aus dem Gehäuse 26 austritt, eine sichere Verriegelung der Messerkartusche an dem Kartuschensitz 24 gegeben ist. Das Instrument 10 ist nun betriebsbereit. Es können Gewebepartien insbesondere Gefäße von dem Versiegelungsinstrument 18 gefasst und durch Bestromung zwischen den beiden Werkzeugteilen 17, 20 versiegelt werden. Durch Betätigung eines Handhebels 48 der Betätigungseinrichtung 36 wird das Messer dann vermittels des in die Ausnehmung 38 greifenden Mitnehmers 37 durch den Schlitz 22 geschoben, um das versiegelte Gefäß zu durchtrennen.

Das Messer 30 kann dabei etwas flexibel sein, um einer Krümmung des Schlitzes 22 folgen zu können, wenn die Branchen eine seitliche Krümmung aufweisen.

Zum Wechsel der Messerkartusche 25 wird zunächst der Betätigungshebel 48 in Entriegelungsstellung gebracht, so dass der Mitnehmer 37 aus der Ausnehmung 38 ausfährt. Das Messer 30 kann nun bis zu einem Anschlag zurückgezogen werden. In der so eingenommenen Position fluchtet die Ausnehmung 39 wieder mit dem Sperrglied 41, sodass das Messer 30 eine Bewegung des Sperrglieds 41 quer zu dem Messer 30 nicht mehr sperrt. In diesem Zustand kann die Messerkartusche 25 von dem Kartuschensitz 24 abgezogen werden, wobei sich das Sperrglied 41 unter der Wirkung des Federelements 43 in seine Sperrstellung zurückbewegt. Zugleich fährt die Sperrnase 46 aus der Sperröffnung 47 aus, wodurch das Lösen der Messerkartusche 25 möglich ist. Das Federmittel 43 ist dabei optional. Es dient vorwiegend der Unterstützung der Lösebewegung und dazu, das Sperrglied 41 dauerhaft in Sperrstellung zu halten.

Es versteht sich, dass andere Verriegelungsmechanismen möglich sind. Beispielsweise kann das Sperrglied 41 lediglich für das Sperren der Messerbewegung zuständig sein und auf eine Verriegelung der Messerkartusche 25 an dem Kartuschensitz 24 verzichtet werden. Auch ist die Verwendung eines schwenkbeweglichen Sperrglieds möglich, das zum Beispiel mit dem Gehäuse 26 einstückig ausgebildet sein kann.

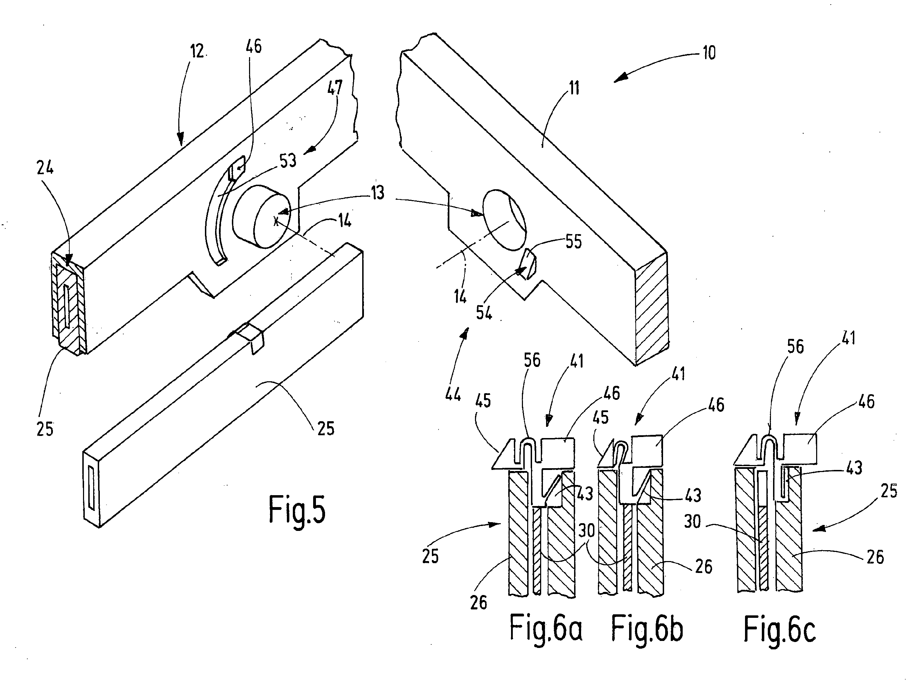

Eine weitere Ausführungsform des Versiegelungsinstruments 10 ist einschließlich der darin zu platzierenden Messerkartusche 25 in Figur 5 in Explosionsdarstellung perspektivisch mit gespreizten Branchen 11, 12 veranschaulicht. Die Branchen 11, 12 sind dabei jeweils zu beiden Seiten ihres Gelenks 13 zur Verbesserung der Übersichtlichkeit abgeschnitten dargestellt. Wie ersichtlich, weist die zweite Branche 12 den Kartuschensitz 24 auf, in dem die Messerkartusche 25 platziert werden kann. Der Kartuschensitz 24 wird zum Beispiel durch eine an einer Schmalseite der Branche 12 offene Nut gebildet, in die die Messerkartusche 25 passt.

Konzentrisch zu der Gelenkachse 14 ist eine bogenförmige Rastnut 53 ausgebildet, die als Sperröffnung 47 dient und eine Begrenzungswand des Kartuschensitzes 24 durchsetzt. Figur 5 veranschaulicht das Sperrglied 41 wie es mit seiner Sperrnase 46 in die Rastnut 53 eingerastet ist.

Die Branche 11 weist einen in die Rastnut 53 passenden Nocken 54 auf, der das Entriegelungselement 44 bildet. Beim Verschwenken der beiden Branchen 11, 12 gegeneinander fährt er in der Rastnut 53 entlang. Der Nocken 54 weist eine Betätigungsfläche 55 auf, die gegen die Gelenkachse 14 geneigt ist.

Das Sperrglied 41 und dessen Funktion geht aus den Figuren 6a bis 6c hervor. Wie erkennbar, weist es eine Sperrnase 46 auf, die durch eine an dem Sperrglied 41 oder auch dem Gehäuse 26 angeordnete oder ausgebildete Federzunge oder ein sonstiges Federmittel 43 in eine Sperrstellung vorgespannt ist, in der das Messer 30 blockiert ist

Zu dem Sperrelement 41 gehört wiederum eine Betätigungsfläche 45 an einem Betätigungsstück, das über ein Federelement 56 mit der Sperrnase 46 verbunden ist. Das Federelement 56 kann mit dem Betätigungselement und/oder der Sperrnase 46 einstückig ausgebildet sein.

Figur 6a veranschaulicht das Sperrglied 41 in Ruheposition. Das Federmittel 43 hält das Sperrglied 41 in einer Sperrposition, in der das Messer 30 gegen Längsbewegungen arretiert ist.

Wird die Messerkartusche 25 in den Kartuschensitz 24 eingeführt, drängt eine Flanke des Kartuschensitzes zugleich die Betätigungsfläche 44 in die in Figur 6b veranschaulichte Position. Jedoch verhindert die an der gegenüber liegenden Wand des Kartuschensitzes 24 entlang laufende Sperrnase 46 eine Bewegung des Sperrglieds 41 in Freigabeposition, sodass das Messer 30 gemäß Figur 6b gesperrt bleibt.

Sobald die Messerkartusche 25 in ihrer Sollposition angekommen ist, erreicht die Sperrnase 46 die Rastnut 53, sodass sie unter der Wirkung des sich nun wieder entspannenden Federelements 56 in die Rastnut 53 einrastet. In dieser Stellung verriegelt das Sperrglied 41 die Messerkartusche 25 an dem Kartuschensitz 24.

Die beiden Branchen 11, 12 können beim Einsatz des Instruments am Patienten gegeneinander geschwenkt werden, ohne dass der Nocken 54 die Sperrnase 46 berührt. Ist das Versiegelungselement 10 ein offenchirurgisches Instrument, ähnlich wie Figur 1 veranschaulicht, ist dabei die Sperrnut 53 und somit der Leergang des Nockens 54 in der Sperrnut 53 mindestens so groß, dass ein Bediener auch mit größter anzunehmender Hand die Handgriffe 15, 23 um mindestens eine Spanne (Abstand zwischen Mittelfinger und Daumen) voneinander weg bewegen kann, ohne dass der Nocken 54 an der Sperrnase 46 anläuft. Werden die beiden Branchen 11, 12 jedoch weiter voneinander weg geschwenkt, wirkt der Nocken 54 mit seiner Betätigungsfläche 55 auf die Sperrnase 46 ein und drängt diese nach innen. Dies gelingt nur bei zurückgezogenem, also innerhalb des Gehäuses 26 stehendem Messer 30. Das Sperrglied 41 der Messerkartusche 25 gerät dadurch aus der Stellung nach Figur 6c in die Stellung nach Figur 6b, womit das Messer 30 arretiert und die Messerkartusche 25 in dem Kartuschensitz 24 zur Herausnahme freigegeben ist. Sie kann nun entnommen werden.

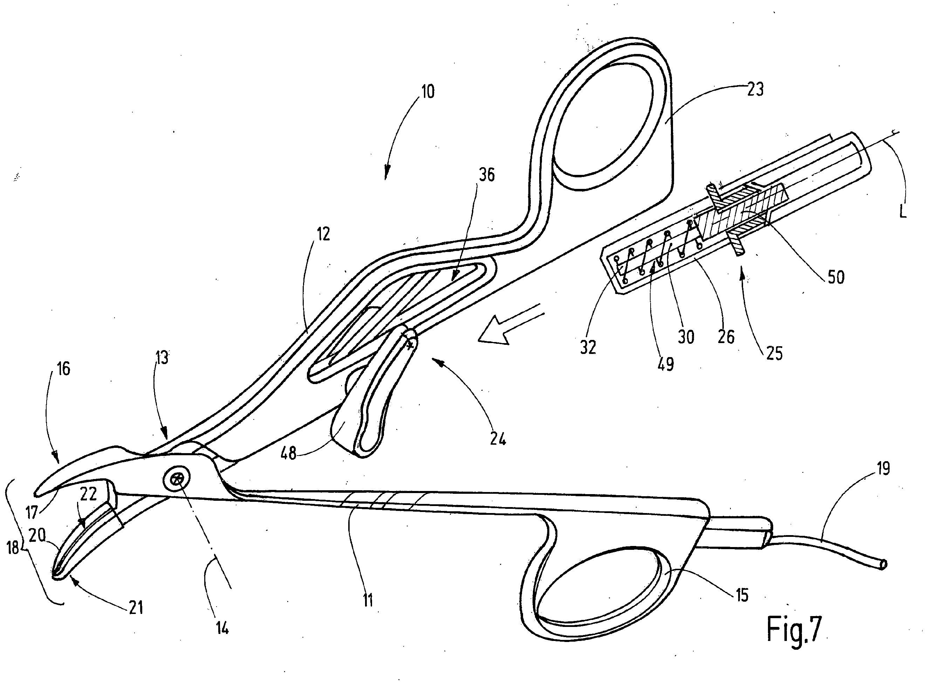

Weiter ist es möglich, auf einen Verriegelungsmechanismus zu verzichten und das Messer 30 zum Beispiel durch energiespeichernde Elemente, wie einen Magnet oder ein Federelement 48, in sicherer Stellung zu halten, wie es in Figur 7 angedeutet ist. Das Messer 30 kann dort in einem Sockel 49 gehalten sein, der aus dem Gehäuse 26 ragende Fortsätze aufweist. Die Fortsätze können genutzt werden, um das Messer 30 manuell und temporär zu Inspektionszwecken aus dem Gehäuse 26 herauszuschieben. Außerdem können die Fortsätze als Ansatz für die Betätigungseinrichtung 36 dienen. Ansonsten gilt für die Ausführungsform des Instruments 10 nach Figur 7 die Beschreibung zu der Ausführungsform nach Figur 1 unter Zugrundelegung der bereits eingeführten Bezugszeichen entsprechend.

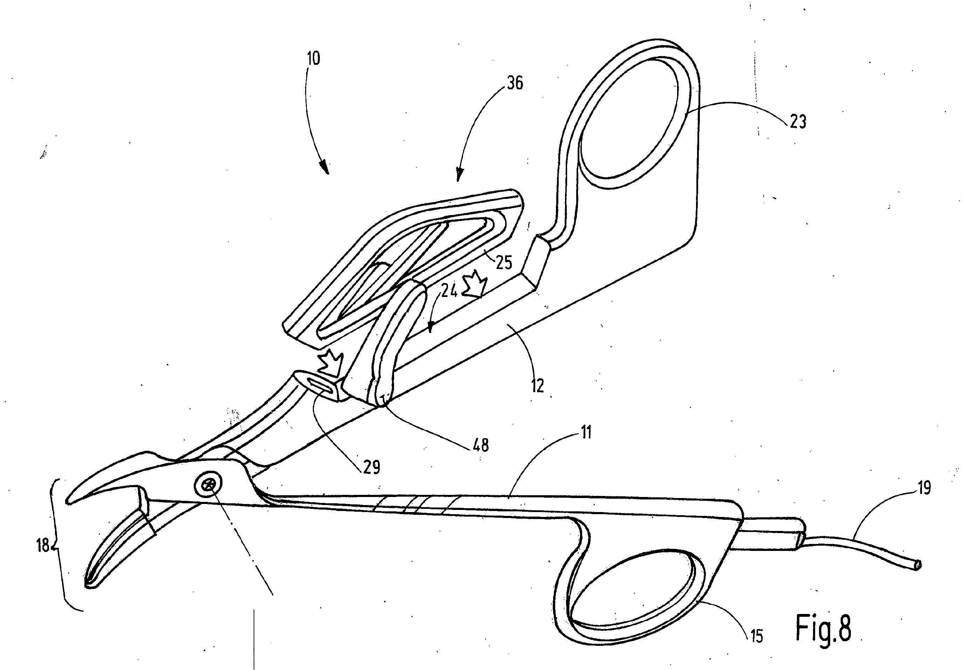

Bei einer weiteren abgewandelten Ausführungsform des Instruments 10 kann, wie es Figur 8 veranschaulicht, die Betätigungseinrichtung 36 auch mit der Messerkartusche 25 zu einer Baueinheit vereinigt sein. Der Kartuschensitz 24 nimmt die aus Betätigungseinheit 36 und Messerkartusche bestehende Einheit auf, wobei geeignete Rast- und Positionierungsmittel vorgesehenen sind, um den in Figur 8 nicht weiter veranschaulichten Messerkanal 28 mit dem Messerkanal 29 in Fluchtung zu bringen.

Auch bei dieser Ausführungsform kann ein Sperrmechanismus vorgesehen sein, der die Betätigung des Betätigungshebels 48 und somit ein Ausfahren des Messers erst gestattet, wenn die Messerkartusche 25 ordnungsgemäß mit dem Kartuschensitz 25 verbunden ist. Auch hier gilt ansonsten die vorstehende Beschreibung zu den übrigen Ausführungsformen der Erfindung ergänzend.

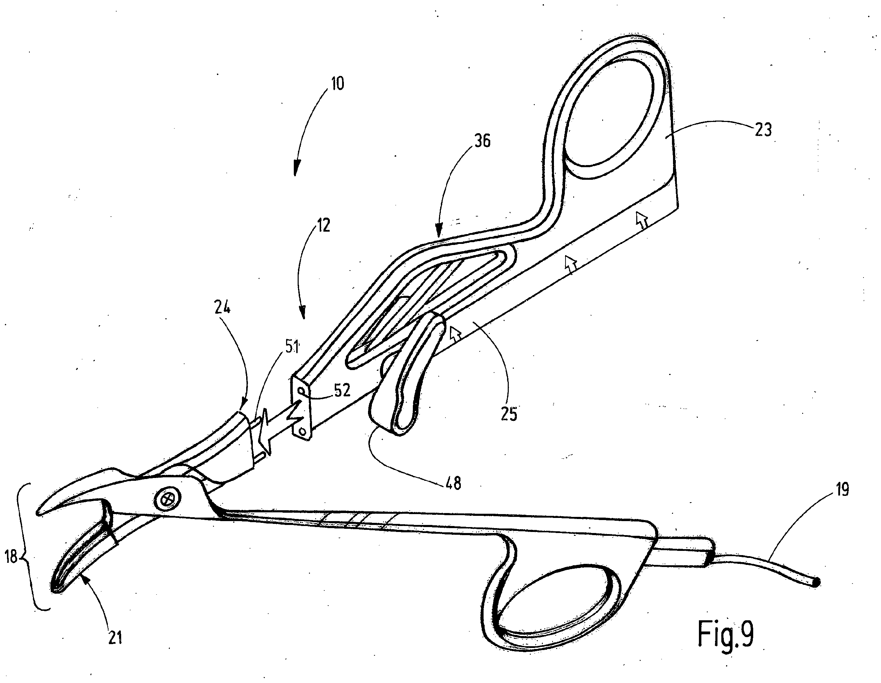

Figur 9 veranschaulicht eine weiter abgewandelte Ausführungsform des Instruments 10, bei der die Messerkartusche 25 nicht nur die Betätigungseinrichtung 36, sondern zusätzlich noch den Handgriff 23 enthält. Der Kartuschensitz 24 ist somit zugleich eine Verbindungsstelle, an der die Kraft zum Schließen des Versiegelungswerkzeugs 18 von dem proximalen Teil der Branche 12 auf den distalen Teil 21 derselben übertragen wird. Die Messerkartusche 25 bildet somit den proximalen Teil der Branche 12.

Auch bei dieser Ausführungsform des Instruments 10 kann wiederum ein Verriegelungsmechanismus vorgesehen sein, der die Betätigung des Messers, d.h., ein Ausfahren desselben aus der Messerkartusche 25, erst dann freigibt, wenn die Messerkartusche 25 mit dem Kartuschensitz 24 ordnungsgemäß verbunden ist. Dazu können beispielsweise ein oder mehrere Entriegelungsstifte 50 vorgesehen sein, die an dem Kartuschensitz 24 vorgesehen sind und in entsprechende Öffnungen 51 einfahren, um die Bewegung des Messers zu entsperren und/oder eine Kopplung zwischen der Betätigungseinrichtung 36 und dem Messer herzustellen.

Das erfindungsgemäße Konzept sieht ein Instrument 10 vor, das einen Kartuschensitz 24 für einen Messerkartusche 25 aufweist. Die Messerkartusche 25 hält bei Nichtgebrauch, insbesondere wenn die Messerkartusche 25 nicht in das Versiegelungsinstrument 10 eingesetzt ist, ein Messer 30 vorzugsweise in geschützter sicherer Position, in der die Schneide 32 des Messers 30 verlässlich innerhalb des Gehäuses 26 der Messerkartusche 25 gehalten ist.

Dieses Konzept führt zu einer erhöhten Bediensicherheit beim medizinischen Personal.

### Bezugszeichen:

- 10: Versiegelungsinstrument
- 11: erste Branche
- 12: zweite Branche
- 13: Gelenk
- 14: Schenkachse
- 15: Handgriff an der ersten Branche 11
- 16: distales Ende der ersten Branche 11
- 17: erster Werkzeugteil
- 18: Versiegelungswerkzeug
- 19: Kabel
- 20: zweiter Werkzeugteil
- 21: distales Ende der zweiten Branche 12
- 22: Schlitz
- 23: Handgriff an der zweiten Branche 12
- 24: Kartuschensitz
- 25: Messerkartusche
- 26: Gehäuse der Messerkartusche 25
- 27: Innenraum des Gehäuses 26
- 28: Messerkanal des Gehäuses 26
- 29: Messerkanal der zweiten Branche 12
- 30: Messer
- 31: stumpfer Abschnitt des Messers 30
- 32: Schneide des Messers 30
- L: Längs- und Verschieberichtung des Messers 30
- 33, 34: Rastmittel
- 35: Ausschnitt
- 36: Betätigungsvorrichtung
- 37: Mitnehmer
- 38, 39: Ausnehmungen in einer Kante des Messers 30
- 40: Verriegelungsmechanismus
- 41: Sperrglied
- 42: Ausnehmung in dem Sperrglied
- 43: Federmittel
- 44: Entriegelungselement
- 45: Betätigungsfläche
- 46: Sperrnase
- 47: Sperröffnung
- 48: Betätigungshebel
- 49: Federelement
- 50: Sockel
- 51: Entriegelungsstift
- 52: Öffnung
- 53: Rastnut
- 54: Nocken
- 55: Betätigungsfläche
- 56: Federelement

## Patentansprüche

1. Chirurgisches Versiegelungsinstrument (10), insbesondere zur Versiegelung und Durchtrennung von Gefäßen, mit einer ersten Branche (11), die an ihrem distalen Ende (16) ein erstes Werkzeugteil (17) eines Versiegelungswerkzeugs (18) aufweist,
mit einer zweiten Branche (12), die bezüglich der ersten Branche (11) schwenkbar gelagert ist und die an ihrem distalen Ende (21) ein zweites Werkzeugteil (20) des Versiegelungswerkzeugs (18) aufweist,
mit einem Kartuschensitz (24), der zur Aufnahme einer Messerkartusche (25) eingerichtet ist,
mit einer von dem Kartuschensitz (24) abnehmbaren Messerkartusche (25), die an dem Kartuschensitz (24) angebracht ist, wobei die Messerkartusche (25) aufweist:
ein Gehäuse (26), das einen Innenraum (27) umschließend ausgebildet ist,
ein Messer (30), das mindestens eine Schneidkante (32) aufweist und das eine Kupplungseinrichtung (38) zum Anschluss an eine Betätigungseinrichtung (36) aufweist,
wobei das Messer (30) in der Messerkartusche (25) beweglich angeordnet ist, um aus einer ersten Position, in der die Schneidkante (32) in dem Innenraum (27) angeordnet ist, in eine zweite Position verschoben zu werden, in der die Schneidkante (32) außerhalb der Messerkartusche (25) steht,
**dadurch gekennzeichnet, dass** die Messerkartusche (25) der einzige von dem Instrument lösbare Teil ist, wobei die Messerkartusche (25) und die Werkzeugteile (17, 20) voneinander getrennte, nicht fest miteinander verbundene Baugruppen sind.

2. Versiegelungsinstrument (10) nach Anspruch 1, **dadurch gekennzeichnet, dass** die Messerkartusche (25) einen Verriegelungsmechanismus (40) aufweist, der zwischen dem Gehäuse (26) und dem Messer (30) wirkend angeordnet ist.

3. Versiegelungsinstrument (10) nach Anspruch 2, **dadurch gekennzeichnet, dass** der Verriegelungsmechanismus (40) zur Entriegelung eine Betätigungsfläche (45) aufweist.

4. Versiegelungsinstrument (10) nach einem der Ansprüche 1 bis 3, **dadurch gekennzeichnet, dass** die Messerkartusche (25) einen Verriegelungsmechanismus (40) zur Arretierung des Messers (30) in seiner ersten Position aufweist.

5. Versiegelungsinstrument (10) nach Anspruch 4, **dadurch gekennzeichnet, dass** der Verriegelungsmechanismus (40) ein an oder in dem Gehäuse (26) beweglich gelagertes Sperrglied (41) aufweist, dem eine an dem Messer (30) ausgebildete Sperrstruktur (39) zugeordnet ist.

6. Versiegelungsinstrument nach Anspruch 5, **dadurch gekennzeichnet, dass** das Sperrglied (41) mit einer an dem Kartuschensitz (24) oder der Betätigungseinrichtung (36) ausgebildeten Entriegelungsstruktur (44) zusammenwirkend ausgebildet ist.

7. Versiegelungsinstrument nach einem der vorstehenden Ansprüche 4 bis 6, **dadurch gekennzeichnet, dass** der Verriegelungsmechanismus (40) zur Arretierung der Messerkartusche (25) an dem Kartuschensitz (24) eingerichtet ist.

8. Versiegelungsinstrument einem der vorstehenden Ansprüche 4 bis 7, **dadurch gekennzeichnet, dass** der Verriegelungsmechanismus (40) als Wechselsperre ausgebildet ist, deren Sperrglied (41) in einer ersten Stellung das Messer (30) in der Messerkartusche (25) und in einer zweiten Stellung die Messerkartusche (25) an dem Kartuschensitz (24) arretierend ausgebildet ist.

9. Versiegelungsinstrument nach einem der vorstehenden Ansprüche, **dadurch gekennzeichnet, dass** die Messerkartusche (25) einen Messerkanal (28) aufweist, der mit einem in dem Instrument (10) ausgebildeten Messerkanal (29) fluchtend angeordnet ist, wenn die Messerkartusche (25) an dem Kartuschensitz (24) befestigt ist.

10. Versiegelungsinstrument nach einem der vorstehenden Ansprüche, **dadurch gekennzeichnet, dass** die Messerkartusche (25) eine Messereinzugsvorrichtung aufweist.

11. Versiegelungsinstrument nach Anspruch 10, **dadurch gekennzeichnet, dass** die Messereinzugsvorrichtung ein Federelement (49) ist, das das Messer (30) auf eine Position hin vorspannend angeordnet ist, in der die Schneide (32) des Messers (30) in dem Innenraum (27) des Gehäuses (26) steht.

12. Versiegelungsinstrument nach einem der vorstehenden Ansprüche, **dadurch gekennzeichnet, dass** die Messerkartusche (25) den proximalen Abschnitt der zweiten Branche (12) bildend ausgebildet ist und einen zweiten Handgriff (23) aufweist.

13. Versiegelungsinstrument nach einem der vorstehenden Ansprüche, **dadurch gekennzeichnet, dass** das Messer (30) eine Kupplungsstruktur (38) zum Anschluss eines Mitnehmers (37) der Betätigungseinrichtung (36) aufweist.

## Claims

1. Surgical sealing instrument (10), particularly for sealing and cutting of vessels,
having a first jaw (11) that comprises a first tool part (17) of a sealing tool (18) at its distal end (16),
having a second jaw (12) that is pivotably supported relative to the first jaw (11) and that comprises a second tool part (20) of the sealing tool (18) at its distal end (21),
having a cartridge seat (24) that is configured for reception of a knife cartridge (25),
having a knife cartridge (25), which is attached to cartridge seat (24), being removable from the cartridge seat (24), wherein the knife cartridge (25) comprises:
a housing (26) that encloses an inner space (27),
a knife (30) that comprises at least one cutting edge (32) and that comprises a coupling device (38) for connection to an operating device (36),
wherein the knife (30) is movably arranged in the knife cartridge (25) in order to be shifted from a first position in which the cutting edge (32) is arranged in the inner space (27) into a second position in which the cutting edge (32) is outside of the knife cartridge (25),
**characterized in that** the knife cartridge (25) is the only part that can be removed from the instrument, wherein the knife cartridge (25) and the tool parts (17, 20) are assemblies that are separate from each other and are not rigidly connected with each other.

2. Sealing instrument (10) according to claim 1, **characterized in that** the knife cartridge (25) comprises a locking mechanism (40) that is arranged to be effective between the housing (26) and the knife (30).

3. Sealing instrument (10) according to claim 2, **characterized in that** the locking mechanism (40) comprises an actuating surface (45) for unlocking.

4. Sealing instrument (10) according to any of the claims 1 to 3, **characterized in that** the knife cartridge (25) comprises a locking mechanism (40) for locking the knife (30) in its first position.

5. Sealing instrument (10) according to claim 4, **characterized in that** the locking mechanism (40) comprises a blocking element (41) movably supported at or in the housing (26), wherein a blocking structure (39) formed at the knife (30) is assigned to the blocking element (41).

6. Sealing instrument according to claim 5, **characterized in that** the blocking element (41) is configured to cooperate with an unlocking structure (44) formed at the cartridge seat (24) or the operating device (36).

7. Sealing instrument according to one of the preceding claims 4 to 6, **characterized in that** the locking mechanism (40) is configured for locking the knife cartridge (25) at the cartridge seat (24).

8. Sealing instrument according to one of the preceding claims 4 to 7, **characterized in that** the locking mechanism (40) is configured as reciprocal interlocking, the blocking element (41) thereof is configured to lock the knife (30) in the knife cartridge (25) in a first position and the knife cartridge (25) at the cartridge seat (24) in a second position.

9. Sealing instrument according to any of the preceding claims, **characterized in that** the knife cartridge (25) comprises a knife channel (28) that is arranged in alignment with a knife channel (29) formed in the instrument (10), if the knife cartridge (25) is attached to the cartridge seat (24) .

10. Sealing instrument according to any of the preceding claims, **characterized in that** the knife cartridge (25) comprises a knife retraction device.

11. Sealing instrument according to claim 10, **characterized in that** the knife retraction device is a spring element (49) that is arranged to pretension the knife (30) toward a position in which the blade (32) of the knife (30) is in the inner space (27) of housing (26).

12. Sealing instrument according to any of the preceding claims, **characterized in that** the knife cartridge (25) is configured to form the proximal section of the second jaw (12) and comprises a second handle (23).

13. Sealing instrument according to any of the preceding claims, **characterized in that** the knife (30) comprises a coupling structure (38) for connection with a tappet (37) of the operating device (36).

## Revendications

1. Instrument de scellement chirurgical (10), destiné en particulier au scellement et à la coupe de vaisseaux,
comprenant une première branche (11) qui présente à son extrémité distale (16) une première partie d'outil (17) d'un outil de scellement (18),
comprenant une deuxième branche (12) qui est montée avec possibilité de pivotement par rapport à la première branche (11) et qui présente à son extrémité distale (21) une deuxième partie d'outil (20) de l'outil de scellement (18),
comprenant un siège de cartouche (24) qui est conçu pour accueillir une cartouche de lame (25),
comprenant une cartouche de lame (25) qui peut être retirée du siège de cartouche (24) et qui est fixée au siège de cartouche (24), la cartouche de lame (25) présentant :
un boîtier (26) qui est réalisé de manière à entourer un espace intérieur (27),
une lame (30) qui présente au moins une arête de coupe (32) et qui comporte un dispositif d'accouplement (38) en vue du raccordement à un dispositif d'actionnement (36),
la lame (30) étant disposée de façon mobile dans la cartouche de lame (25), aux fins de pouvoir être glissée d'une première position, dans laquelle l'arête de coupe (32) est logée dans l'espace intérieur (27), dans une deuxième position dans laquelle l'arête de coupe (32) se trouve à l'extérieur de la cartouche de lame (25),
**caractérisé en ce que** la cartouche de lame (25) est la seule partie pouvant être séparée de l'instrument, la cartouche de lame (25) et les parties d'outil (17, 20) étant des modules séparés qui ne sont pas liés solidement l'un à l'autre.

2. Instrument de scellement (10) selon la revendication 1, **caractérisé en ce que** la cartouche de lame (25) présente un mécanisme de verrouillage (40) qui est disposé de manière à agir entre le boîtier (26) et la lame (30).

3. Instrument de scellement (10) selon la revendication 2, **caractérisé en ce que** le mécanisme de verrouillage (40) présente une surface d'actionnement (45) en vue du déverrouillage.

4. Instrument de scellement (10) selon l'une des revendications 1 à 3, **caractérisé en ce que** la cartouche de lame (25) présente un mécanisme de verrouillage (40) destiné à bloquer la lame (30) dans sa première position.

5. Instrument de scellement (10) selon la revendication 4, **caractérisé en ce que** le mécanisme de verrouillage (40) présente un élément de blocage (41) qui est monté de façon mobile sur ou dans le boîtier (26) et auquel est associée une structure de blocage (39) réalisée sur la lame (30).

6. Instrument de scellement selon la revendication 5, **caractérisé en ce que** l'élément de blocage (41) est conçu pour coopérer avec une structure de déverrouillage (44) réalisée sur le siège de cartouche (24) ou le dispositif d'actionnement (36).

7. Instrument de scellement selon l'une des revendications précédentes 4 à 6, **caractérisé en ce que** le mécanisme de verrouillage (40) est conçu pour le blocage de la cartouche de lame (25) sur le siège de cartouche (24).

8. Instrument de scellement selon l'une des revendications précédentes 4 à 7, **caractérisé en ce que** le mécanisme de verrouillage (40) est réalisé comme dispositif de blocage alternatif dont l'élément de blocage (41) est conçu pour bloquer la lame (30) dans la cartouche de lame (25), dans une première position, et la cartouche de lame (25) sur le siège de cartouche (24), dans une deuxième position.

9. Instrument de scellement selon l'une des revendications précédentes, **caractérisé en ce que** la cartouche de lame (25) présente un conduit de lame (28) qui est disposé de manière à être aligné avec un conduit de lame (29) réalisé dans l'instrument (10), lorsque la cartouche de lame (25) est fixée au siège de cartouche (24).

10. Instrument de scellement selon l'une des revendications précédentes, **caractérisé en ce que** la cartouche de lame (25) présente un dispositif de rétractation de lame.

11. Instrument de scellement selon la revendication 10, **caractérisé en ce que** le dispositif de rétractation de lame est un élément à ressort (49) qui est disposé de manière à exercer une précontrainte sur la lame (30), en direction d'une position dans laquelle le tranchant (32) de la lame (30) se trouve dans l'espace intérieur (27) du boîtier (26).

12. Instrument de scellement selon l'une des revendications précédentes, **caractérisé en ce que** la cartouche de lame (25) est réalisée de manière à former la partie proximale de la deuxième branche (12) et présente un deuxième manche (23).

13. Instrument de scellement selon l'une des revendications précédentes, **caractérisé en ce que** la lame (30) présente une structure d'accouplement (38) destinée au raccordement d'un élément d'entraînement (37) du dispositif d'actionnement (36).
